# EUROPEAN PATENT SPECIFICATION

(11) **EP 4 415 669 B1**
(45) Date of publication and mention of the grant of the patent: **25.02.2026**
(21) Application number: 22801886.7
(22) Date of filing: 13.10.2022
(51) Int. Cl.: A61F 13/511, A61F 13/512

(54) **MULTI-LAYERED MATERIAL, IN PARTICULAR FOR HYGIENE /SANITARY PRODUCTS, AND MANUFACTURING METHOD FOR MAKING SUCH A MULTI - LAYERED MATERIAL**
MEHRSCHICHTIGES MATERIAL, INSBESONDERE FÜR HYGIENE-/SANITÄRPRODUKTE, UND HERSTELLUNGSVERFAHREN ZUR HERSTELLUNG EINES SOLCHEN MEHRSCHICHTIGEN MATERIALS
MATÉRIAU MULTICOUCHE, EN PARTICULIER POUR PRODUITS D'HYGIÈNE/SANITAIRES, ET PROCÉDÉ DE FABRICATION D'UN TEL MATÉRIAU MULTICOUCHE

(30) Priority: 15.10.2021 IT 202100026495
(43) Date of publication of application: 21.08.2024
(73) Proprietor: FITESA SULMONA S.R.L., 20060 Trezzano Rosa (MI) (IT)
(72) Inventor: ANGELI, Pietro, 67039 SULMONA (AQ) (IT)
(74) Representative: Studio Torta S.p.A.
(86) International application number: PCT/IB2022/059818
(87) International publication number: WO 2023/062576

(56) References cited:
- US-A- 5 370 764
- US-A1- 2018 071 156
- US-A1- 2021 186 777
- US-B2- 7 993 317

## Description

### Cross-Reference to Related Applications

This Patent Application claims priority from Italian Patent Application No. 102021000026495 filed on October 15.

### Technical Field

The present invention concerns a multi-layered material to be used, in particular, in hygiene/sanitary products.

### Prior Art

As is known, webs that include non-woven fabrics are widely used in many industrial sectors. One sector in which said webs are particularly widespread is, for example, the production of hygiene/sanitary products.

A typical application is the one relative to nappies and sanitary pads. In general, nappies and sanitary pads are formed essentially of four elements which, starting from the one in contact with the skin, are called: topsheet, sublayer, core and backsheet.

With specific reference to the topsheet, the latter must be constructed so that the liquids released by the user can rapidly pass through it, but it must then act as a barrier between the user's skin and the inner part of the nappy/sanitary pad which is designed to capture and retain the liquids.

To guarantee permeability towards the inner part of the nappy/sanitary pad, the top-sheet must be through-perforated, perpendicular to the outer surface thereof, providing a series of channels so that the liquids to be absorbed can pass through said channels in a substantially unidirectional manner.

At the same time, the top-sheet, given its direct contact with the skin, must be relatively soft to the touch, so as not to irritate the skin and so as to create if possible a pleasant tactile sensation.

In the context of these applications, the need is particularly felt for a top-sheet which is an optimal compromise between the requirement for a high level of softness and a pleasant feeling to the touch and the requirement for a high unidirectional permeability.

Many known solutions have tackled the need to obtain a high level of softness, for example the solution described in EP2353809, in the name of the holder of the current patent application.

By means of the production method described in this document, the outer surface of a non-woven fabric is shaped so as to obtain a series of first areas, in which through holes are provided, and a series of second areas, without holes, arranged around the first areas, embossed with respect to the latter and therefore thicker and softer.

The need is felt to perfect this type of product, especially as regards the softness and pleasant feeling to the touch along the surface which, in use, will be in contact with the user's skin.

The object of the present invention is therefore to meet the above-mentioned needs in a simple inexpensive manner, developing a perforated multi-layered material that is particularly soft and pleasant to the touch and guarantees an optimal compromise with the permeability requirement described above.

US2021186777 discloses an apparatus for making a multilayer absorbent element apt to be incorporated in a sanitary article. The apparatus has an embossing roller bearing a plurality of protruding elements, a pierced roller bearing a plurality of protruding elements and cavities, and a unit of cohesion and drilling. The configuration of the apparatus is such that, after a first layer of sheet material is deformed by the embossing roll and the perforated roller continues to slide on the pierced roller, a second layer of sheet material is superimposed. The first and second layer of sheet material are then carried to the joining and piercing unit in which, at the intermediate zones of the first layer, it forms a plurality of holes passing through the first and second layer and joins them near or at the plurality of holes.

US7993317 discloses an absorbent article having a topsheet and an absorbent core in facing relationship with the topsheet. The topsheet has a central region, an inner intermediate region, an outer intermediate region, and an edge region, wherein the inner intermediate region is between the central region and the outer intermediate region and the outer intermediate region is between the inner intermediate region and the edge region. The central region texture, inner intermediate region texture, outer intermediate region texture, and edge region texture differ from one another. At least one of the central region, inner intermediate region, outer intermediate region, and edge region comprises tufted fibers. The central region is on the longitudinal centerline of the absorbent article.

US2018071156 discloses a liquid permeable substrate for an absorbent article. The substrate comprises a nonwoven hydrophobic layer. The substrate comprises a plurality of recesses, a plurality of projections, and a plurality of land areas. The land areas surround at least a majority of the plurality of projections and a plurality of the recesses. The plurality of recesses, the plurality of projections, and the plurality of land areas together form a first three-dimensional surface on a first side of the substrate and a second three-dimensional surface on a second side of the substrate. A hydrophilic material is positioned only proximate to at least some of the apertures.

US5370764 discloses a film laminated material wherein a first film layer comprises a thin film of a thermoplastic material and a second fibrous layer comprises a plurality of staple fibers or continuous filaments of two or more thermoplastic or other materials bonded together in a spaced apart bonding pattern having apertures formed therein to form a film laminate having improved liquid distribution and management properties as well as enhanced comfort and softness when placed in contact with human skin.

### Summary of the Invention

The above-mentioned object is achieved by a multi-layered material, in particular for hygiene/sanitary products, and by a relative production method, as claimed in the attached claims.

### Brief Description of the Drawings

For a better understanding of the present invention, at least one preferred embodiment is described below by way of nonlimiting example and with reference to the attached drawings, in which:
- figure 1 illustrates, in perspective and in a simplified manner, a preferred embodiment of the multi-layered material according to the present invention;
- figure 2 is a photograph that shows an outer surface of the multi-layered material of figure 1;
- figures 3 and 4 are photographs (by scanning electron microscope) that show, on an enlarged scale, details of the multi-layered material of figure 2;
- figures 5 and 6 are a photograph (by scanning electron microscope) and a diagram respectively that show, on an enlarged scale, a cross section of the multi-layered material of the preceding figures;
- figure 7 schematically illustrates a preferred method for producing the multi-layered material of the preceding figures, by means of two counter-rotating rollers;
- figure 8 illustrates, on an enlarged scale and schematically, a cross section obtained according to the line of section VIII-VIII of figure 7;
- figures 9 and 10 show a configuration of the outer surface of the counter-rotating rollers of figures 7 and 8; and
- figures 11 to 14 illustrate some possible examples as variations of the configuration of figure 9.

### Detailed Disclosure

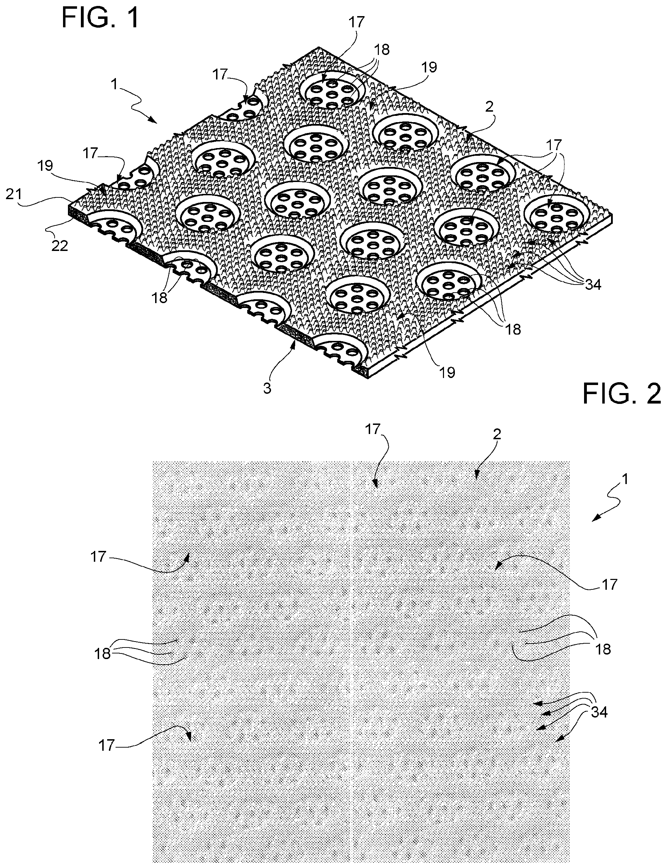

In figure 1, the reference number 1 indicates a perforated web (partially illustrated) which according to one aspect of the present invention is defined by a multi-layered material. The multi-layered material 1 has two surfaces 2 and 3, opposite each other: the surface 2 comprises a plurality of recessed areas 17, in which a plurality of holes 18 are provided, passing through the multi-layered material 1. The recessed areas 17 are distributed, preferably in a uniform manner, along the surface 2 and are spaced from one another by means of at least one embossed area 19, comprising portions that are arranged alongside, for example all around, the recessed areas 17. The embossed area 19, along the surface 2, protrudes with respect to the recessed areas 17: in other words, in the embossed area 19, the thickness of the multi-layered material 1 (understood as the distance between the surfaces 2 and 3, perpendicular to the latter) is greater then the thickness at the recessed areas 17.

In the preferred example illustrated, the multi-layered material 1 comprises two layers 21 and 22: the layer 21 defines the surface 2 and is a film made of plastic material, microperforated at least in the embossed area 19, while the layer 22 defines the surface 3 and is a non-woven fabric, bonded by lamination to the layer 21. According to variations not illustrated, the multi-layered material 1 can be laminated so as to have at least one additional layer, arranged between the layers 21 and 22 or arranged along the surface 3.

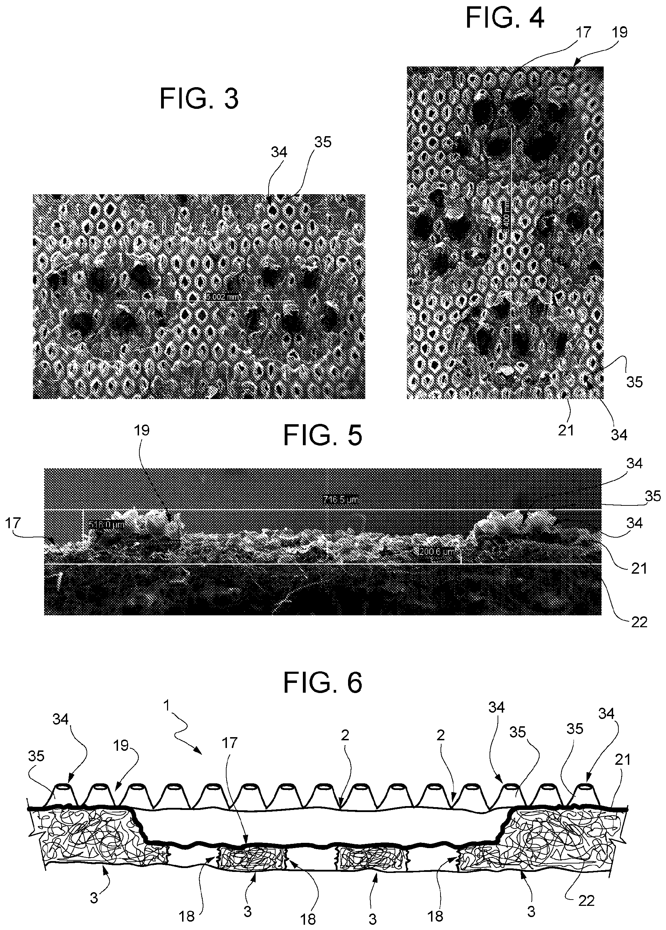

In figures 1 and 5 where a schematic cross section of the multi-layered material 1 can be seen, the thicknesses are not shown to scale. In the example of figure 6, the thicknesses identified (by scanning electron microscope) are approximately 0.72 mm in the embossed area 19 and approximately 0.20 mm in the recessed areas 17, therefore the embossed area 19 protrudes by approximately 0.52 mm. More generally, the overall thickness of the multi-layered material 1 is comprised between 2 and 5 times the thickness at the recessed areas 17; preferably, the thickness of the multi-layered material 1 is comprised between 0.4 and 1 mm, while the thickness at the recessed areas 17 is comprised between 0.1 and 0.6 mm.

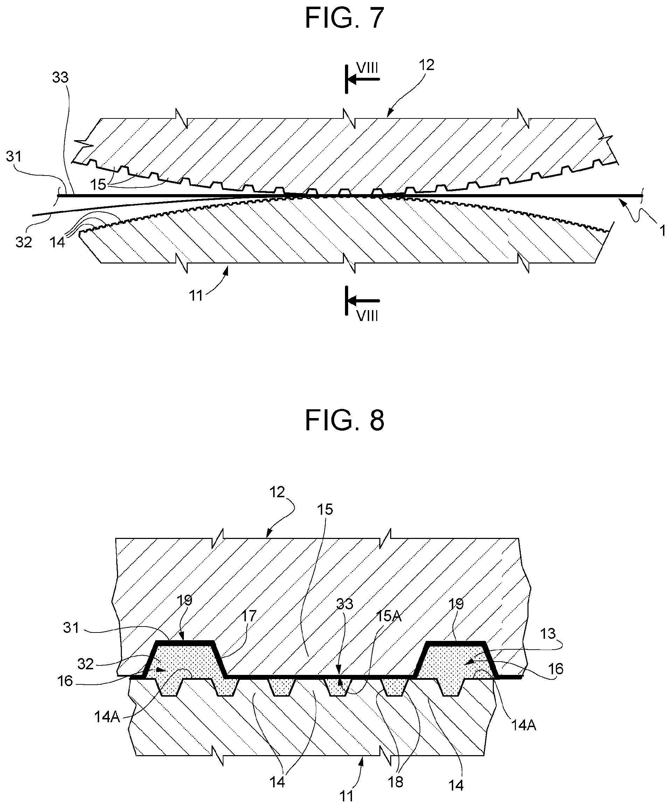

Preferably, the areas 17,19 and the holes 18 are formed by means of the process described in EP2353809 and illustrated in figures 7 and 8, in one single station and by means of one single operation, thanks to the use of a pair of counter-rotating rollers, indicated by the reference numbers 11 and 12. During said operation, the rollers 11 and 12 are pressed towards each other in a radial direction and, immediately before the pressure area, define an inlet towards which at least two bands or sheets 31 and 32 are fed, which will define the layers 21 and 22 respectively at the end of the process. In the pressure area between the rollers 11 and 12, the sheets 31 and 32 advance and are arranged one above the other in order to undergo, together, the shaping step in order to produce the recessed areas 17, and the perforation step in order to produce the holes 18.

Preferably, the roller 11 is heated. The operating temperature is a function, for example, of the type of multi-layered material 1 and the work speed. Again by way of example, said temperature is comprised between 70°C and 170°C (typically 120°C). Preferably, also the roller 12 is heated, for example to a temperature comprised between 70°C and 170°C.

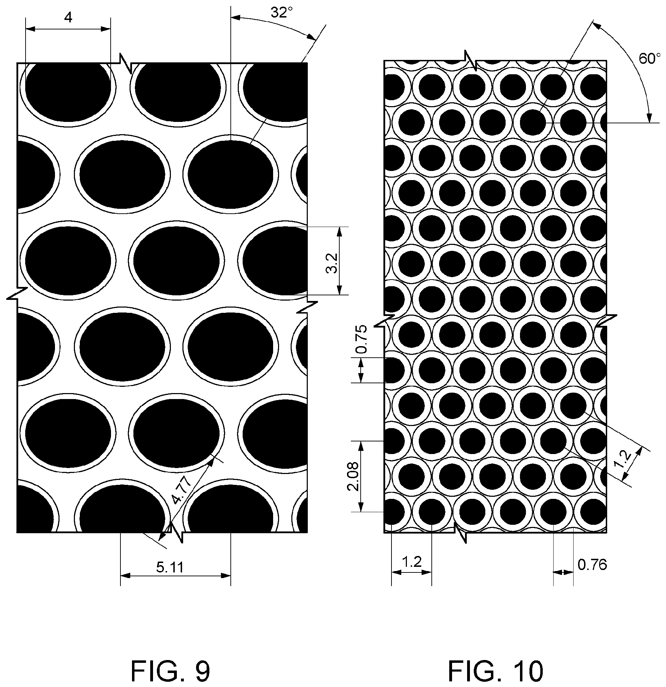

The roller 11 has, on its outer cylindrical surface, a plurality of protrusions 14 which protrude in radial directions with respect to the axis of the roller 11. Preferably, the protrusions 14 are distributed uniformly along the cylindrical extension of the roller 11, so as to define a given geometric pattern. For example, as can be seen in figure 10, the protrusions 14 are aligned with one another along a series of circumferences, lying on planes perpendicular to the axis of the roller 11, and are arranged at a constant pitch along each of said circumferences (the value of said pitch, in general, is called "longitudinal pitch"); more preferably, the protrusions 14 are offset in a longitudinal (namely circumferential) direction with respect to the adjacent protrusions 14, for example they are offset by half a pitch. The longitudinal direction is usually indicated by the initials M.D.

As can be seen in figure 8 (corresponding to a section plane on which the axis of the roller 11 lies), for example, the section of each protrusion 14 is trapezoidal, more precisely an equilateral trapezium, tapered outwards, whereby the protrusions 14 have a truncated cone shape. The protrusions 14 terminate radially in a face 14A which lies along the outer cylindrical surface of the roller 11, and which makes contact on the sheet 32 (namely on the surface 3 of the multi-layered material 1) to perforate both the sheets 31 and 32 and form the holes 18. For example, the faces 14A and therefore the holes 18 have a substantially circular perimeter (figure 10).

At the same time, the roller 12 has a plurality of appendages 15 which protrude in a radial direction, with respect to the axis of the roller 12. Each of the appendages 15 defines a forming head with an end face 15A which lies along the outer cylindrical surface of the roller 12 and makes contact on a surface 33 of the sheet 31 so as to form the recessed areas 17.

Also in this case, preferably, the appendages 15 are distributed uniformly along the cylindrical extension of the roller 12, so as to define a given geometric pattern or geometric configuration. For example, as shown in figure 9, the appendages 15 are aligned with one another along a series of circumferences, lying on planes perpendicular to the axis of the roller 12, and are arranged at a constant longitudinal pitch; more preferably, the appendages 15 are offset along the longitudinal direction with respect to the appendages 15 of the adjacent row, for example they are offset by half a pitch.

For example, as can be seen in figure 8, also the appendages 15 have a truncated cone shape, tapered outwards. In the preferred example of figure 10, the bases of said truncated cone and therefore the faces 15A have an elliptic shape, with larger diameter arranged parallel to the axis of the roller 12. Consequently also the recessed areas 17 have, in plan view, a substantially elliptic shape (figures 2-4). According to variations not illustrated, the faces 15A of the appendages 15 and therefore the recessed areas can have a circular shape (in this case, the transverse dimension and the longitudinal dimension are identical) or a polygonal shape.

Again with reference to figure 8, along the periphery of the roller 11, the appendages 15 are surrounded by depressions 16, so that in practice they form "islands" separated from one another. In other words, the outer cylindrical surface of the roller 12 is formed of the faces 15A and of these depressions 16.

Following this operation, therefore, the dimensions and the shape of the recessed areas 17 of the multi-layered material 1 correspond substantially to the faces 15A of the roller 12. At the same time, the holes 18 have dimensions and shape which substantially correspond to the faces 14A of the roller 11. The term "substantially" must be understood in the sense that there will never be a perfect correspondence, in terms of dimensions and shape, between
- the faces 15A of the appendages 15 and the recessed areas 17, and
- the faces 14A of the protrusions 14 and the holes 18,
due to the deformability of the materials, the sliding between rollers and the material treated, the working tolerances, the forming parameters (pressure, temperature, relative speed between the rollers), etc. For example, comparing figures 3 and 4 with figure 9, between the recessed areas 17 a transverse pitch (orthogonal to the longitudinal feed direction and parallel to the roller axes) is measured of approximately 5.00 mm and a longitudinal pitch (along the longitudinal feed direction, tangential to the rollers) of approximately 6.40 mm, instead of the pitches of the appendages 15 equal to 5.11 mm and 8.05 mm respectively. As regards the holes 18, in particular, the mean diameter measured can differ even by 30% with respect to the diameter of the faces 14A; moreover, some of the holes 18 are arranged along the perimeter edge of the recessed areas 17 (in fact, some faces 14A of the roller 11, during the perforation, rest and slide on the perimeter edge of the faces 15A), so that they present an incomplete area with respect to the holes 18 which are completely included inside the perimeter edge of the recessed areas 17.

The pitch (longitudinal and transverse) between the protrusions 14 must obviously be much smaller than the one between the appendages 15; in this way, contact between the faces 15A of the appendages 15 and the faces 14A of the protrusions 14 is guaranteed. In other words, the faces 15A of the appendages 15 define respective abutment areas for the protrusions 14. In particular, the ratio between the longitudinal pitches (and/or between the transverse pitches) of the faces 15A and 14A is preferably between 1.5 and 10. Calibrating this value, advantageously, optimal permeability can be obtained in relation to optimal shaping in terms of thickness and softness.

From a different point of view, this compromise between optimal permeability and optimal feeling of softness can be obtained by calibrating the mean number of holes 18 present for each of the recessed areas 17 (namely the mean number of protrusions 14 that cooperate with each appendage 15, in the pressure area), and/or the number per cm2 of the recessed areas 17 (as a mean value on the surface 2, which corresponds approximately to the number per cm2 of the appendages 15, as a mean value on the outer cylindrical surface of the roller 12), and/or the mean diameter of the holes 18 (which corresponds substantially to the mean diameter of the faces 14A, barring deviations that can reach 30%, as mentioned above).

In this regard, preferably: the number of holes 18 for each recessed area 17 is comprised between 3 and 12, and more preferably between 4 and 10; the number per cm2 of the recessed areas 17 (as mean surface value) is comprised between 1 and 8; the mean diameter of the holes 18 is comprised between 0.2 and 0.8 mm.

In the particular example illustrated in figure 9, in addition to the above-mentioned transverse and longitudinal pitches, the transverse dimension of each face 15A is equal to approximately 4.0 mm, and the longitudinal dimension is equal to approximately 3.2 mm. It is expedient for the faces 15A (and therefore the recessed areas 17) to have a minimum surface area, defined for example by a minimum dimension (transverse and/or longitudinal) of 1.5 mm.

Again in the preferred example, the number per cm2 of the appendages 15 is on average equal to approximately 4.9 and the surface per cm2 of the faces 15A on average is equal to approximately 0.489 (namely approximately 49%). In general, as already mentioned above, the number per cm2 of the appendages 15 (and therefore of the recessed areas 17) is preferably comprised between 1 and 8, on average. Alternatively, or in combination with these ranges, the ratio between the pitch (longitudinal and/or transverse) of the faces 15A and their dimension (longitudinal and/or transverse) is comprised between 1.1 and 4: these parameters are calibrated to allow the multi-layered material 1 to provide a high level of thickness and softness.

As regards the faces 14A on the roller 11, with reference to the particular example in figure 10, the transverse pitch is equal to approximately 1.2 mm, the transverse dimension is equal to approximately 0.76 mm, the longitudinal pitch is approximately 2.08 mm and the longitudinal dimension is equal to approximately 0.75 mm.

More in general, it is expedient for the faces 14A to have a minimum surface area, defined for example by a minimum diameter of 0.2 mm, corresponding to the minimum diameter at the holes 18 to guarantee a minimum level of permeability to liquids through the multi-layered material 1 (from the surface 2 towards the surface 3).

Again in the example illustrated, the number per cm2 of the protrusions 14 is, on average, equal to approximately 80, and the surface per cm2 of the faces 14A is equal to approximately 0.359 (namely approximately 36%). These values, however, can change so as to determine the desired number of holes 18 for each recessed area 17, and determine the passage area of each hole 18.

As mentioned above, the rollers 11,12 are pressed radially towards each other; for example, the linear pressure between the two rollers is comprised between 40 N/mm and 200 N/mm. The holes 18 of the multi-layered material 1 are obtained from the protrusions 14 by abrasion, during the pressure against the appendages 15, due to relative sliding with respect to the latter. For said purpose, the roller 11 rotates at a higher speed than the roller 12, so that the faces 14A slide on the sheet 32 (namely on the surface 3 of the multi-layered material 1). For example, the speeds of the two rollers 11, 12 are 70 m/min and 50 m/min respectively (hence the relative linear sliding is equal to approximately 30%).

At the same time, as explained above, the appendages 15 leave an impression which is substantially funnel-shaped and defines the recessed areas 17 of the multi-layered material 1, while the material of the sheet 32 tends to move up the depressions 16 between the appendages 15 to define the embossed area 19 of the multi-layered material 1.

From figure 8, it is evident that the holes 18 are obtained solely at the recessed areas 17, and not at the embossed area 19. The depressions 16 perform a relief function for the material treated: in fact, the non-woven fabric that has filled the depressions 16 tends to "swell" and is therefore softer in the embossed area 19.

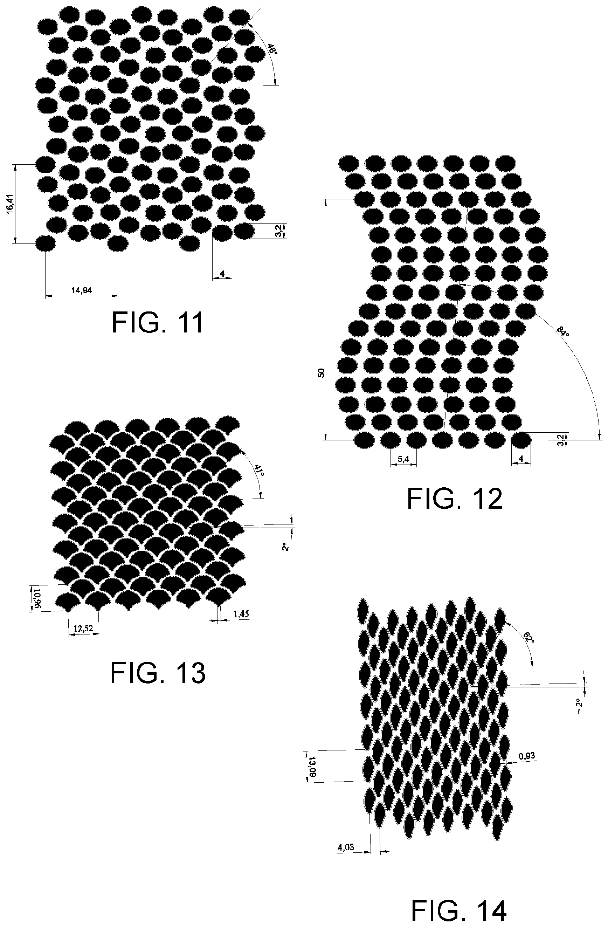

Obviously, the configurations of the rollers 11,12 can be different from the example described above. For example, it is possible to verify the number and/or the pitches of the protrusions 14, and/or the shape of the faces 15A, and/or the pattern of the appendages 15, and/or the pitches of said appendages 15, etc.... For example, the appendages 15 (and therefore the recessed areas 17) could be distributed without any pattern, namely at random, as in figure 11; or have a wave pattern as in figure 12 (namely the appendages 15 are aligned with one another along a series of waves, along the outer cylindrical surface of the roller 12, instead of being arranged along circumferences), or even form waves which are longitudinally continuous (not illustrated); or have perimeter and pitches different from those indicated above, as in the example of figure 13 (where the number per cm2 of the appendages 15 is equal to approximately 1.43 and the surface per cm2 of the faces 15A is equal to approximately 66.4%) or in the example of figure 14 (where the number per cm2 of the appendages 15 is equal to approximately 1.89 and the surface per cm2 of the faces 15A is equal to approximately 53.6%).

Furthermore, the formation of the recessed areas 17 and creation of the holes 18 could be carried out by means of techniques different from the preferred one illustrated and described above: for example, they could be carried out in two successive operations and/or by means of devices that do not cooperate with each another (contrary to the rollers 11,12).

According to an aspect of the present invention, the sheet 31 is defined by a plastic film which is micro-perforated substantially throughout its extension, and preferably in a uniform manner. For example, the plastic material of this film can be polyethylene-based.

As mentioned above, the surface 33 of the sheet 31 makes contact with the faces 15A of the roller 12 so as to define, at the end of the process, the surface 2 of the layer 21.

The sheet 32, which will define the layer 22, is a non-woven fabric, such as a cohesive and substantially uniform layer of fibres, which can be synthetic or natural, for example a layer of polypropylene fibres, or a layer of bi-component fibres (polypropylene-polyethylene or polypropylene-polyester, for example), or a layer of natural fibres like cotton or viscose. In the example considered, as mentioned above, the sheet 32 contacts the roller 11 so as to define, at the end of the process, the layer 22 and the surface 3 of the multi-layered material 1.

During the production process, the sheets 31 and 32, and therefore the layers 21 and 22, are bonded to each other by lamination, in particular hot lamination, namely by passing between two heated cylinders radially pressed towards each other. In particular, an intermediate layer can be provided, but preferably they are laminated directly to each other. In a first embodiment, not illustrated, the sheets 31 and 32 are made to adhere by lamination in a preliminary station, before arriving at the inlet of the rollers 11,12. In other words, a continuous web is pre-formed by lamination of the two sheets 31,32 and then fed towards the rollers 11,12. In the preferred embodiment illustrated in figure 7, on the other hand, the sheets 31 and 32 are coupled to each other by lamination by the rollers 11 and 12 (at least one of which is heated), without any preliminary lamination. In this case, the sheets 31 and 32 are fed towards the inlet of the rollers 11 and 12 separately: for example, they come into contact with each other at said inlet.

If the rollers 11,12 also carry out the lamination, the layers 21 and 22 are bonded to each other only at the recessed areas 17, due to the pressure and heat exerted at the appendages 15. Preferably, the area of the recessed areas 17 (and therefore the area of bonding between the layers 21 and 22) is at least equal to 30%.

In this case, therefore, the lamination for coupling the sheets 31 and 32, the forming to create the recessed areas 17, and the perforation to create the holes 18, are carried out in one single operation by a pair of counter-rotating rollers defined, in particular, by the rollers 11,12 described above by way of example.

With reference to figures 3 to 5, the layer 21 has a plurality of micro-openings or micro-holes 34, derived from the fact that also the sheet 31 has the same micro-holes or micro-openings (figure 8). On the sheet 31, they are distributed uniformly along the surface 33, in a manner not illustrated. At the same time, preferably, the sheet 32 is a substantially uniform material made of fibres, without holes or micro-holes, before forming the recessed areas 17 and creating the holes 18.

According to a preferred aspect of the present invention, the edge of the micro-holes 34 is defined by a respective cusp 35, which protrudes along the surface 2 with respect to the remaining part of the layer 21, towards the outside (namely in opposite orientation to the layer 22, and opposite to the direction in which the sheet 31 is pressed to form the recessed areas 17 by means of the impression of the appendages 15). In practice, the cusps 35 are deformed portions of the film, substantially in the shape of a truncated cone and with a small end opening at the tip. The passages in the cusps 35 are therefore tapered as far as said end opening which defines the diameter of the microholes 34.

The micro-holes on the sheet 31 are formed in a preliminary phase by means of known techniques, in a manner not illustrated, in this specific case by means of a technique called "vacuum perforation": a continuous plastic film, preferably uniform, advances in contact with a rotating cylinder, which has a microperforated outer surface, subjected to underpressure by means of a cavity inside the cylinder: due to the pressure difference between the inside and the outside of the cylinder, the film is micro-perforated.

Alternatively, for example, a needle perforation technique can be used, although in this case the edges around the micro-holes 34 protrude less than the cusps 35 formed by underpressure.

As a further alternative, an abrasion perforation technique can be used. Also in this case, the micro-holes 34 do not have the cusps 35 described above; in place of the cusps 35, the edge or perimeter of the micro-holes 34 has a lip (not illustrated) that stays attached to the remaining part of the film and protrudes along the surface 33 and, therefore, the surface 2 towards the outside, so that it performs a function similar to the one performed by the cusps 35.

As a further alternative, not illustrated, the sheet 31 is without micro-holes 34, and is obtained by plastic deformation so that it presents micro-swellings, micro-bubbles or microprotrusions which are distributed along the surface 33 (preferably in a uniform manner), protrude with respect to said surface 33 and will define corresponding micro-portions protruding along the surface 2 of the layer 21 (at least in the embossed areas, as will be described below), with the same function as the cusps 35. In other words, it is possible to obtain the same effect as the cusps 35 by means of a deformation that does not perforate (in a through manner) the sheet 31.

With reference to figures 3 and 4, the micro-holes 34 with the corresponding cusps 35 are easily recognisable in the embossed area 19, but tend to be partially closed at the recessed areas 17. This phenomenon is due essentially to the compression used to form the recessed areas 17; in fact, the pressure exerted by the appendages 15 on the surface 33 of the sheet 31 to form the recessed areas 17 acts directly on the tips of the cusps 35 and, compressing them towards the roller 11, tends to flatten said tips with at least partial closure of the width of their end openings.

At the same time, at the depressions 16 and, therefore, the embossed area 19, the cusps 35 are not significantly pressed by the rollers 11,12, hence the micro-holes 34 and the cusps 35 on the layer 21 correspond substantially to those already provided on the sheet 31 of origin.

As can be seen in figures 3 and 4, the perimeter of the microholes 34 is fairly irregular. On average, each micro-hole 34 has a passage area comprised between approximately 0.032 and 0.070 mm2, which corresponds to a diameter which, on average, is comprised between 0.17 and 0.30 mm. In general, by the term "micro-hole", we mean an opening that passes through the sheet 31 and therefore the layer 21 (without involving the sheet 32 and the layer 22) and, on average, has a smaller diameter than that of the holes 18.

Preferably, the density of the micro-holes 34 through the sheet 31 (and therefore through the layer 21, at least at the embossed area 19), on average, is comprised between 30 and 100 mesh (by the term mesh we mean unit per linear inch).

Preferably, the sheet 31 has a gram weight comprised between 6 and 25, and/or a thickness which is comprised between 0.25 and 0.45 mm (measured by thickness gauge).

Preferably, the sheet 32 has a gram weight comprised between 6 and 30 g/m2, and/or a thickness comprised between 0.08 and 1 mm (measured by thickness gauge).

The initial thicknesses of the sheets 31 and 32 are, in fact, of little importance in determining the final thickness of the multi-layered material 1, due to the process which they undergo (pressures, lamination, etc...), and due to the fact that the fibre material of the non-woven fabric tends to "swell" in the embossed area 19.

In the multi-layered material 1 produced according to the teachings of the present invention, a certain feeling of softness is provided by the shaping or three-dimensionality of the surface 2, due to the distribution of the recessed areas 17 within the embossed area 19, in both longitudinal and transverse direction. This feeling is improved due to the cusps 35 or any other type of protruding micro-portions of film which, in the specific example, are provided along the edges of the microholes 35: these small protrusions tend to create the feeling of a velvety surface to the touch. In other words, the cusps 35 help to reduce the so-called "plasticky feeling" and, therefore, make the surface 2 more pleasant and soft in contact with the skin.

The addition of a film (layer 21) to the layer 22 of non-woven fabric does not compromise the passage and permeation of liquids towards the surface 3, since said liquids flow regularly through the holes 18 (which are made through both the layers 21 and 22). In this regard, the recessed areas 17 tend to perform the function of a funnel for the holes 18 to channel the liquids as they pass through the multi-layered material 1.

The micro-holes 34, provided in the preferred solution illustrated, tend to improve this passage of liquids.

At the same time, the layer 21 can contribute to improving the unidirectionality of said permeation, as it can perform the function of an additional barrier for retaining the liquids, since it tends to prevent reflux of the liquids back towards the surface 2. In practice, the micro-holes 34 do not affect said reflux, since the open area of the sheet 31 is relatively limited (for example, around 20% of the total area). Moreover, preferably, the micro-holes 34 are at least partially closed at the recessed areas 17, hence this passage is further reduced in said areas.

From the above it is therefore evident that the multi-layered material 1 constitutes an optimal compromise between the need for unidirectional permeability and the need for softness, and that it is an improvement on the known solutions which provide a single layer of non-woven fabric. In particular, this compromise is due to the simultaneous presence and combination of the cusps 35, the holes 18 and the recessed areas 17: the calibration of the dimensions and quantity (or density) of these three elements provides an optimal result.

Moreover, the proposed production method is particularly simple and advantageous, since it minimizes the operations and times for forming the multi-layered material 1, in view of the possibility of carrying out together the lamination for bonding the sheets 31 and 32, forming of the surface 2, and perforation for forming the holes 18. In particular, the equipment defined by the rollers 11 and 12, used according to the preferred embodiment described above, can be identical, in practice, to the equipment already described and illustrated in the patent EP2353809, with consequent saving in time and costs in setting up the production lines.

From the above it is evident that the production method and the multi-layered material 1 described above with reference to the attached figures can be subject to modifications and variations, which do not depart from the protective scope defined by the attached claims.

In particular, as already mentioned above, the steps of lamination, forming of the recessed areas 17 and creation of the holes 18 could be performed using techniques different from the preferred one which has been indicated by way of example, and if necessary one after the other, instead of being carried out together at the pressure area between the rollers 11,12.

## Claims

1. A multi-layered material (1), in particular for hygiene/sanitary products, the multi-layered material comprising:
- an outer surface (2) shaped so as to comprise:
a) a plurality of recessed areas (17) and
b) at least one embossed area (19) which is arranged alongside said recessed areas (17) and protrudes with respect to said recessed areas (17) at said outer surface (2) so as to define a thickness of the multi-layered material greater than the one at said recessed areas (17);
- a plurality of through holes (18), formed only through said recessed areas (17);
- a first layer and a second layer (21,22) coupled to one another, whereby said through holes (18) pass through both said first and said second layers (21,22);
wherein said second layer (22) is a non-woven fabric; and
wherein said first layer (21) defines said outer surface (2) and is a film made of plastic material comprising a plurality of protruding micro-portions (35), that are provided on at least said embossed area (19) and protrude outwards with respect to the remaining part of the first layer (21) along said outer surface (2).

2. The multi-layered material according to claim 1, wherein said protruding micro-portions (35) are arranged along the edges of respective micro-holes (34) provided through the first layer (21), at least at said embossed area (19).

3. The multi-layered material according to claim 2, wherein said micro-holes (34) are at least partially closed at said recessed areas (17).

4. The multi-layered material according to claim 2 or 3, wherein said micro-holes (34), at said embossed area (19), have a diameter that, on average, is comprised between 0.1 and 0.30 mm.

5. The multi-layered material according to any one of the preceding claims, wherein the density of said protruding micro-portions (35) is comprised between 30 and 100 mesh.

6. The multi-layered material according to any one of the preceding claims, consisting only of said first and second layers (21,22).

7. The multi-layered material according to any one of the preceding claims, wherein said first and second layers (21,22) are bonded to one another only at said recessed areas (17).

8. The multi-layered material according to any one of the preceding claims, wherein the surface of said recessed areas (17) is at least equal to 30% of said outer surface (2).

9. The multi-layered material according to any one of the preceding claims, wherein the number of through holes (18) for each recessed area (17) is comprised between 3 and 12, in particular between 4 and 10.

10. The multi-layered material according to any one of the preceding claims, wherein the diameter of said through holes (18) is, on average, greater than 0.2 mm.

11. A manufacturing method for making a multi-layered material (1), in particular for hygiene/sanitary products, the method comprising:
- a forming step so as to shape a surface (33) that will define an outer surface (2) of the multi-layered material (1) and obtain:
a) a plurality of recessed areas (17) and
b) at least one embossed area (19) which is arranged alongside said recessed areas (17) and protrudes with respect to said recessed areas (17) so as to define a thickness of the multi-layered material greater than the one at said recessed areas (17);
- a perforating step so as to form a plurality of through holes (18) through said multi-layered material (1) only at said recessed areas (17);
- a laminating step starting from a first and a second sheet (31,32) for subjecting the whole of said first and second sheets (31,32) to the forming and perforating steps;
wherein said second sheet (32) is a non-woven fabric; and
wherein said first sheet (31) defines said surface (33) and is a film made of microperforated plastic material comprising a plurality of protruding micro-portions (35), that protrude with respect to the remaining part of the first sheet along said surface (33) in an opposite direction to said second sheet (32) and are arranged along the edges of respective microholes.

12. The method according to claim 11, wherein said forming and perforating steps are carried out in a single operation by a pair of counter-rotating rollers (11,12).

13. The method according to claim 11 or 12, wherein said laminating step is carried out together with said forming and perforating steps in a single operation by said pair of counter-rotating rollers (11,12).

14. The method according to claim 13, wherein the forming step comprises a compression, which acts on said surface (33) to form said recessed areas (17) and at least partially closes the micro-holes (34) at said recessed areas (17).

## Patentansprüche

1. Mehrschichtiges Material (1), insbesondere für Hygiene-/Sanitärprodukte, wobei das mehrschichtige Material umfasst:
- eine Außenoberfläche (2), die so geformt ist, dass sie Folgendes umfasst:
a) eine Vielzahl von vertieften Bereichen (17) und
b) mindestens einen geprägten Bereich (19), der neben den vertieften Bereichen (17) angeordnet ist und in Bezug auf die vertieften Bereiche (17) an der Außenoberfläche (2) vorsteht, um eine Dicke des mehrschichtigen Materials zu definieren, die größer ist als diejenige an den vertieften Bereichen (17);
- eine Vielzahl von Durchgangslöchern (18), die nur durch die vertieften Bereiche (17) gebildet sind;
- eine erste Schicht und eine zweite Schicht (21, 22), die miteinander gekoppelt sind, wodurch die Durchgangslöcher (18) sowohl durch die erste als auch durch die zweite Schicht (21,22) verlaufen;
wobei die zweite Schicht (22) ein Vliesstoff ist; und wobei die erste Schicht (21) die Außenoberfläche (2) definiert und ein Film aus Kunststoffmaterial ist, der eine Vielzahl von vorstehenden Mikroabschnitten (35) umfasst, die auf mindestens dem geprägten Bereich (19) bereitgestellt sind und in Bezug auf den verbleibenden Teil der ersten Schicht (21) entlang der Außenoberfläche (2) nach außen vorstehen.

2. Mehrschichtiges Material nach Anspruch 1, wobei die vorstehenden Mikroabschnitte (35) entlang der Ränder jeweiliger Mikrolöcher (34), die durch die erste Schicht (21) bereitgestellt sind, zumindest an dem geprägten Bereich (19) angeordnet sind.

3. Mehrschichtiges Material nach Anspruch 2, wobei die Mikrolöcher (34) an den vertieften Bereichen (17) zumindest teilweise geschlossen sind.

4. Mehrschichtiges Material nach Anspruch 2 oder 3,
wobei die Mikrolöcher (34) an dem geprägten Bereich (19) einen Durchmesser aufweisen, der im Durchschnitt zwischen 0,1 und 0,30 mm liegt.

5. Mehrschichtiges Material nach einem der vorhergehenden Ansprüche, wobei die Dichte der vorstehenden Mikroabschnitte (35) zwischen 30 und 100 Maschen liegt.

6. Mehrschichtiges Material nach einem der vorhergehenden Ansprüche, das nur aus der ersten und der zweiten Schicht (21,22) besteht.

7. Mehrschichtiges Material nach einem der vorhergehenden Ansprüche, wobei die erste und die zweite Schicht (21,22) nur an den vertieften Bereichen (17) miteinander geklebt sind.

8. Mehrschichtiges Material nach einem der vorhergehenden Ansprüche, wobei die Oberfläche der vertieften Bereiche mindestens gleich 30 % der Außenoberfläche (2) ist.

9. Mehrschichtiges Material nach einem der vorhergehenden Ansprüche, wobei die Anzahl der Durchgangslöcher (18) für jeden vertieften Bereich (17) zwischen 3 und 12, insbesondere zwischen 4 und 10, liegt.

10. Mehrschichtiges Material nach einem der vorhergehenden Ansprüche, wobei der Durchmesser der Durchgangslöcher (18) im Durchschnitt größer als 0,2 mm ist.

11. Fertigungsverfahren zur Herstellung eines mehrschichtigen Materials (1), insbesondere für Hygiene-/Sanitärprodukte, wobei das Verfahren umfasst:
- einen Bildungsschritt, um eine Oberfläche (33) zu formen, die eine Außenoberfläche (2) des mehrschichtigen Materials (1) definieren wird und um Folgendes zu erhalten:
a) eine Vielzahl von vertieften Bereichen (17) und
b) mindestens einen geprägten Bereich (19), der neben den vertieften Bereichen (17) angeordnet ist und in Bezug auf die vertieften Bereiche (17) vorsteht, um eine Dicke des mehrschichtigen Materials zu definieren, die größer ist als diejenige an den vertieften Bereichen (17);
- einen Perforationsschritt, um eine Vielzahl von Durchgangslöchern (18) durch das mehrschichtige Material (1) nur an den vertieften Bereichen (17) zu bilden;
- einen Laminierschritt, der von einer ersten und einer zweiten Folie (31,32) ausgeht, um die gesamte erste und zweite Folie (31,32) den Bildungs- und Perforationsschritten zu unterziehen;
wobei die zweite Folie (32) ein Vliesstoff ist; und wobei die erste Folie (31) die Oberfläche (33) definiert und ein Film aus mikroperforiertem Kunststoffmaterial ist, der eine Vielzahl von vorstehenden Mikroabschnitten (35) umfasst, die in Bezug auf den verbleibenden Teil der ersten Folie entlang der Oberfläche (33) in eine entgegengesetzte Richtung zu der zweiten Folie (32) vorstehen und entlang der Ränder jeweiliger Mikrolöcher angeordnet sind.

12. Verfahren nach Anspruch 11, wobei die Bildungs- und Perforationsschritte in einem einzigen Arbeitsgang durch ein Paar gegenläufig rotierender Walzen (11,12) durchgeführt werden.

13. Verfahren nach Anspruch 11 oder 12, wobei der Laminierschritt zusammen mit den Bildungs- und Perforationsschritten in einem einzigen Arbeitsgang durch das Paar gegenläufig rotierender Walzen (11,12) durchgeführt wird.

14. Verfahren nach Anspruch 13, wobei der Bildungsschritt eine Kompression umfasst, die auf die Oberfläche (33) wirkt, um die vertieften Bereiche (17) zu bilden, und die Mikrolöcher (34) an den vertieften Bereichen (17) zumindest teilweise verschließt.

## Revendications

1. Matériau multicouche (1) en particulier pour des produits hygiéniques/sanitaires, le matériau multicouche comprenant :
- une surface extérieure (2) formée de manière à comprendre :
a) une pluralité de zones en retrait (17) et
b) au moins une zone en relief (19) qui est disposée à côté desdites zones en retrait (17) et fait saillie par rapport auxdites zones en retrait (17) au niveau de ladite surface extérieure (2) de manière à définir une épaisseur du matériau multicouche supérieure à celle desdites zones en retrait (17) ;
- une pluralité de trous traversants (18), formés uniquement à travers lesdites zones en retrait (17) ;
- une première couche et une seconde couche (21, 22) couplées l'une à l'autre, de sorte que lesdits trous traversants (18) passent à la fois à travers ladite première et ladite seconde couche (21, 22) ;
dans lequel ladite seconde couche (22) est un tissu non tissé ; et dans lequel ladite première couche (21) définit ladite surface extérieure (2) et est un film en matière plastique comprenant une pluralité de micro-portions saillantes (35), qui sont présentes sur au moins ladite zone en relief (19) et font saillie vers l'extérieur par rapport à la partie restante de la première couche (21) le long de ladite surface extérieure (2).

2. Matériau multicouche selon la revendication 1, dans lequel lesdites micro-portions saillantes (35) sont disposées le long des bords de micro-trous respectifs (34) ménagés à travers la première couche (21), au moins au niveau de ladite zone en relief (19).

3. Matériau multicouche selon la revendication 2, dans lequel lesdits micro-trous (34) sont au moins partiellement fermés au niveau desdites zones en retrait (17).

4. Matériau multicouche selon la revendication 2 ou 3,
dans lequel lesdits micro-trous (34), au niveau de ladite zone en relief (19), ont un diamètre qui, en moyenne, est compris entre 0,1 et 0,30 mm.

5. Matériau multicouche selon l'une quelconque des revendications précédentes, dans lequel la densité desdites micro-portions saillantes (35) est comprise entre 30 et 100 mesh.

6. Matériau multicouche selon l'une quelconque des revendications précédentes, constitué uniquement desdites première et seconde couches (21,22).

7. Matériau multicouche selon l'une quelconque des revendications précédentes, dans lequel lesdites première et seconde couches (21, 22) ne sont collées l'une à l'autre qu'au niveau desdites zones en retrait (17).

8. Matériau multicouche selon l'une quelconque des revendications précédentes, dans lequel la surface desdites zones en retrait est au moins égale à 30 % de ladite surface extérieure (2).

9. Matériau multicouche selon l'une quelconque des revendications précédentes, dans lequel le nombre de trous traversants (18) pour chaque zone en retrait (17) est compris entre 3 et 12, en particulier entre 4 et 10.

10. Matériau multicouche selon l'une quelconque des revendications précédentes, dans lequel le diamètre desdits trous traversants (18) est, en moyenne, supérieur à 0,2 mm.

11. Procédé de fabrication pour la réalisation d'un matériau multicouche (1), en particulier pour des produits hygiéniques/sanitaires, le procédé comprenant :
- une étape de formage afin de façonner une surface (33) qui définira une surface extérieure (2) du matériau multicouche (1) et d'obtenir :
a) une pluralité de zones en retrait (17) et
b) au moins une zone en relief (19) qui est disposée à côté desdites zones en retrait (17) et fait saillie par rapport auxdites zones en retrait (17) de manière à définir une épaisseur du matériau multicouche supérieure à celle desdites zones en retrait (17) ;
- une étape de perforation afin de former une pluralité de trous traversants (18) à travers ledit matériau multicouche (1) uniquement au niveau desdites zones en retrait (17) ;
- une étape de laminage à partir d'une première et d'une seconde feuille (31,32) pour soumettre l'ensemble desdites première et seconde feuilles (31,32) aux étapes de formage et de perforation ;
dans lequel ladite seconde feuille (32) est un tissu non tissé ; et dans lequel ladite première feuille (31) définit ladite surface (33) et est un film en matière plastique microperforée comprenant une pluralité de micro-portions saillantes (35), qui font saillie par rapport à la partie restante de la première feuille le long de ladite surface (33) dans une direction opposée à ladite seconde feuille (32) et sont disposées le long des bords de micro-trous respectifs.

12. Procédé selon la revendication 11, dans lequel lesdites étapes de formage et de perforation sont réalisées en une seule opération par une paire de rouleaux contrarotatifs (11,12).

13. Procédé selon la revendication 11 ou 12, dans lequel ladite étape de laminage est réalisée en même temps que lesdites étapes de formage et de perforation en une seule opération par ladite paire de rouleaux contrarotatifs (11,12).

14. Procédé selon la revendication 13, dans lequel l'étape de formage comprend une compression, qui agit sur ladite surface (33) pour former lesdites zones en retrait (17) et ferme au moins partiellement les micro-trous (34) au niveau desdites zones en retrait (17).
